# EUROPÄISCHE PATENTANMELDUNG

(11) **EP 0 729 728 A1**
(43) Veröffentlichungstag der Anmeldung: **04.09.1996**
(21) Anmeldenummer: 95890042.5
(22) Anmeldetag: 28.02.1995
(51) Int. Cl.: A61B 5/14

(54) **Blutentnahmegerät**

(71) Anmelder: AVL Medical Instruments AG, CH-8207 Schaffhausen (CH)
(72) Erfinder: Kleinhappl, Erich, Ing., A-8044 Graz (AT)
(74) Vertreter: Krause, Walter, Dr. Dipl.-Ing.

(57) **Zusammenfassung**

Ein Blutentnahmegerät, insbesondere zur Entnahme von arteriellen Blutproben, weist einen Griffteil (1), einen Nadeladapter (2), auf welchem eine Punktionskanüle (3) aufsteckbar ist, sowie eine mit der Punktionskanüle (3) in Verbindung stehenden Probenkapillare (4) auf. Für eine robuste und billige Herstellung wird der Griffteil (1) und die Probenkapillare (4) einstückig als Blasformteil ausgeführt und der Nadeladapter (2) entweder einstückig mitgeformt oder als separater Bauteil hergestellt, welcher in eine in die Probenkapillare (4) mündende Öffnung (2') des Griffteiles (1) einsteckbar ist.

## Beschreibung

Die Erfindung betrifft ein Blutentnahmegerät, insbesondere zur Entnahme von arteriellen Blutproben, mit einem Griffteil, einem Nadeladapter, auf welchem eine Punktionskanüle aufsteckbar ist, sowie einer mit der Punktionskanüle in Verbindung stehenden Probenkapillare, sowie ein Verfahren zur Herstellung eines derartigen Gerätes.

Für diagnostische Zwecke, beispielsweise für die Durchführung der Blutgasanalyse, ist es erforderlich, Proben anaerob aus Arterien zu entnehmen. Es sind dazu eine Reihe von Entnahmevorrichtungen bekannt geworden, die im wesentlichen aus einer Zusammenstellung aus Kolbenspritze und Punktionskanüle in steril verpackter Form bestehen. Die verwendeten Spritzen weisen einen Totraum am Eingangsteil der Spritze auf, der mit einem die Blutgerinnung verhindernden Zusatz, beispielsweise mit Heparin gefüllt sein kann.

Ein Entnahmegerät, welches ohne Kolbenspritze auskommt, ist in der AT-PS 363 169 geoffenbart. Die Blutentnahmevorrichtung weist eine zumindest teilweise durchsichtige Hülle auf, deren geschlossene Vorderwand mit einem Stutzen zur Aufnahme einer Punktionskanüle versehen ist. Dieser Stutzen weist eine in das Innere der Hülle mündende Öffnung auf, in welche eine Glaskapillare eingesteckt werden kann. Die Kapillare dient als Probenbehälter und füllt sich durch den arteriellen Blutdruck. Der Stutzen ist mit einer die Punktionskanüle abdeckenden Schutzkappe versehen, welche vor der Blutentnahme abgenommen wird. Im rückwärtigen Ende der Hülle befindet sich ein Verschlußteil, in welchem das rückwärtige Ende der Probenkapillare steckt. Nach der Punktion kann der Verschlußteil mitsamt der gefüllten Kapillare aus der Hülle entnommen und beispielsweise an ein Analysengerät angesteckt werden. In einer Ausführungsvariante kann der Verschlußteil auch zwei durch einen U-förmigen Kanal verbundene Probenkapillaren aufnehmen. Nachteilig bei dieser Ausführungsvariante ist lediglich die relative große Zahl von Einzelteilen, welche die Produktion verteuert und den Manipulationsaufwand beim Zusammenbau und bei der Verwendung des Blutabnahmegerätes vergrößert. Weiters besteht die Gefahr von Undichtheiten durch Toleranzen der Teile, die zusammengesteckt werden müssen.

Andere auf dem Markt befindliche Blutabnahmevorrichtungen weisen ebenfalls die genannten Mängel auf und sind weiters teilweise durch eine schlechte Handhabung, einen fragilen Aufbau (nicht verwendbar in Rohrpostsystemen) oder durch eine relativ große Durchlässigkeit für z. B. Sauerstoff und CO₂ gekennzeichnet. In derartigen Probenentnahmevorrichtungen können daher Blutproben nur wenige Minuten unverfälscht gelagert werden.

Aufgabe der vorliegenden Erfindung ist es, die genannten Nachteile zu vermeiden und insbesondere ein Blutabnahmegerät vorzuschlagen, welches einfach und robust aufgebaut ist und gleichzeitig ein billiges Herstellungsverfahren zuläßt.

Diese Aufgabe wird erfindungsgemäß dadurch gelöst, daß der Griffteil und die Probenkapillare einstückig als Blasformteil ausgeführt sind und daß der Nadeladapter entweder einstückig mitgeformt ist oder als separater Bauteil in eine in die Probenkapillare mündende Öffnung des Griffteiles einsteckbar ist.

Erfindungsgemäß wird das Blutentnahmegerät aus einem thermoplastisch verformbaren, schlauchförmigen Grundkörper im wesentlichen einstückig als Blasformteil hergestellt, wobei die äußeren Konturen durch ein vorzugsweise zweiteiliges Preßwerkzeug und die Lumen der Probenkapillare und allfälliger weiterer Kanäle durch Blasformen dargestellt werden. Falls auch der Nadeladapter einstückig mitgeformt wird, wird meist eine geringe Nachbearbeitung der nicht gratfrei herstellbaren Paßflächen zum Aufsetzen der Punktionskanüle notwendig sein. Andererseits ist es jedoch auch möglich, den Nadeladapter als separaten Spritzgußteil herzustellen, welcher im vorgesehenen Anschlußbereich des kompakt und einfach ausgeführten Blutentnahmegerätes nicht abnehmbar einrastbar ist.

In einer Weiterbildung der Erfindung ist vorgesehen, daß an das vom Nadeladapter abgewandte Ende der Probenkapillare ein einstückig mitgeformtes, mit einer Entlüftung versehenes Reservoir anschließt, welches als Probenüberlauf dient.

Eine besonders leicht zu handbande Ausführungsvariante der Erfindung zeichnet sich dadurch aus, daß das an die Probenkapillare anschließende Reservoir schlauch- oder röhrchenförmig ausgebildet ist, und in einem wulstförmigen Rand des im wesentlichen plättchenförmigen Griffteiles integriert ist.

Das als Microsampler bezeichnete Blutentnahmegerät wurde zur einfachen Blutabnahme direkt am Patienten konzipiert. Die Blutabnahme erfolgt mittels herkömmlicher, beigepackter Punktionskanüle, welche unmittelbar vor dem Punktieren ausgepackt und aufgesteckt wird. Vorteilhafterweise muß nur die Punktionskanüle steril sein, das Blutentnahmegerät selbst braucht unter normalen Einsatzbedingungen nicht sterilisiert werden. Die Blutprobe wird durch den arteriellen Blutdruck in das Entnahmegerät gepumpt, wobei die Entlüftung durch eine kleine Bohrung im wulstförmigen Rand des Griffteiles erfolgt. Zur größtmöglichen Sicherheit für den Patienten ist das Blutentnahmegerät als Einwegartikel konzipiert, welcher und nach Gebrauch entsorgt wird.

Durch die Verwendung von Plastikmaterial, welches für Blutgase wie O₂ und CO₂ eine möglichst geringe Permeabilität aufweist, können Proben für die Blutgasanalyse beispielsweise bis zu 30 Minuten im Blutentnahmegerät gelagert werden. Weiters kann auch durchsichtiges Material verwendet werden, um den Füllungsgrad der Probenkapillare visuell kontrollieren zu können. Dafür geeignete Materialien sind z. B. Acrylnitril-Methyl-Acrylat-Copolymerisate, PMMA, Polycarbonat, Polyethylen und Polypropylen.

In einer erfindungsgemäßen Ausführungsvariante ist vorgesehen, daß an das vom Nadeladapter abgewandte Ende der Probenkapillare ein einstückig mitgeformtes Reservoir anschließt, welches einen durch Fingedruck verformbaren Bereich zum Füllen und Entleeren der Probenkapillare aufweist. Diese Ausführungsvariante besitzt den Vorteil, daß hier ein völlig geschlossenes System - ohne Entlüftung - realisiert werden kann, wobei eine Pump- und Saugwirkung im Probenkanal über den verformbaren Bereich des Reservoirs erzielt werden kann.

Ohne das erfindungsgemäße Blutentnahmegerät wesentlich zu verteuern oder den Assemblieraufwand zu vergrößern, kann in einer Weiterbildung der Erfindung der Griffteil einen einstückig mitgeformten Nadelprotektor aufweisen, welcher über ein mitgeformtes Drehscharnier, vorzugsweise ein Filmscharnier, am Griffteil befestigt ist, wobei der Nadelprotektor durch seine Formgebung zur sicheren Aufnahme der Punktionskanüle ausgebildet ist.

Eine besonders vorteilhafte Ausführungsvariante sieht dabei vor, daß ein zentraler Stegbereich für die integrale Ausbildung des Nadelprotektors vorgesehen ist, welcher an seinem Umfang über mitgeformte, leicht durchdrückbare Sollbruchstellen und an einem biegsamen Verbindungsstück über das Filmscharnier mit dem Griffteil in Verbindung steht. Der Nadelprotektor wird somit ohne weiteren Aufwand automatisch mitgeformt. Dabei werden durch eine entsprechende Werkzeugauslegung die erforderlichen Sollbruchstellen ausgebildet. Nach Abnahme der Blutprobe wird der Nadelprotektor durch Fingerdruck herausgeklappt und über die Punktionskanüle gedreht. Die Punktionskanüle rastet dabei in einer mitgeformten Nut des Nadelprotektors ein, wobei durch ein Verdrehen der Nadel das Filmscharnier des Protektors bricht und die Nadel mitsamt dem Protektor entsorgt werden kann.

Erfindungsgemäß kann der Griffteil des Blutentnahmegerätes zusätzlich eine einstückig mitgeformte Verschlußkappe aufweisen, welche über ein mitgeformtes Drehscharnier, vorzugsweise ein Filmscharnier, am Griffteil befestigt ist, wobei die Verschlußkappe durch ihre Formgebung zum sicheren Verschluß des Nadeladapters ausgebildet ist. Weiters kann ein weiterer Stegbereich für die integrale Ausbildung der Verschlußkappe vorgesehen sein, deren Ausbildung jener des Nadelprotektors entspricht. Nach Abnahme des Nadelprotektors wird die Verschlußkappe durch Fingerdruck herausgeklappt und auf den Nadeladapter gedrückt. Dadurch wird die Probenkapillare auf der Eingangsseite dicht verschlossen, sodaß während des Transportes keine Blutprobe austreten kann.

Beispielhaft soll nun der Ablauf einer typischen Probennahme skizziert werden:
- Auspacken des Microsamplers aus der Plisterverpackung (jeder Sampler ist z. B. einzeln verpackt).
- Auspacken der beigelegten, sterilen Punktionskanüle.
- Aufschieben der Punktionskanüle auf den Luer-Konus des Microsamplers.
- Entfernen der Schutzkappe der Punktionskanüle.
- Punktion des Patienten bis das erforderliche Probenvolumen gesammelt ist.
- Ausklappen des integrierten Nadelprotektors über die Nadel (dadurch Verletzungs- und Kontaminationsschutz).
- Durch Verdrehen wird der Nadelprotektor mitsamt der Punktionskanüle vom Microsampler entfernt und entsorgt.
- Ausklappen der integrierten Verschlußkappe und Aufstecken auf den Luer-Konus des Microsamplers.
- Beschriftung des Microsamplers zur Identifikation der Probe.
- Transport zum Analysengerät (kann auch mittels Rohrpost erfolgen).
- Eingabe der Probe direkt aus dem Microsampler in das Analysengerät.
- Der Microsampler kann nun entsorgt werden.
- Probenanalyse.

Eine Weiterbildung der Erfindung sieht vor, daß weitere Stegbereiche vorgesehen sind, welche eine für die direkte Beschriftung des Blutentnahmegerätes geeignete, vorzugsweise aufgerauhte Oberfläche, bzw. welche eine für die Beklebung mit Etiketten oder maschinenlesbaren Codierungen geeignete, vorzugsweise glatte Oberfläche aufweisen.

Schließlich ist erfindungsgemäß vorgesehen, daß im adapterseitigen Abschnitt des Griffteiles durch eine Eindellung des wulstförmigen Randes eine Griffmulde zur sicheren Handhabung des Blutentnahmegerätes vorgesehen ist. In diesem Bereich kann vorteilhafterweise auch das druckverformbare Reservoir zum Einsaugen und Entleeren der Probenkapillare angeordnet sein.

Im Rahmen des Herstellungsprozesses kann die Innenoberfläche des Blutentnahmegerätes mit einer Heparinlösung gespült werden. Vorzugsweise verwendet man dazu in destilliertem Wasser gelöstes Heparin.

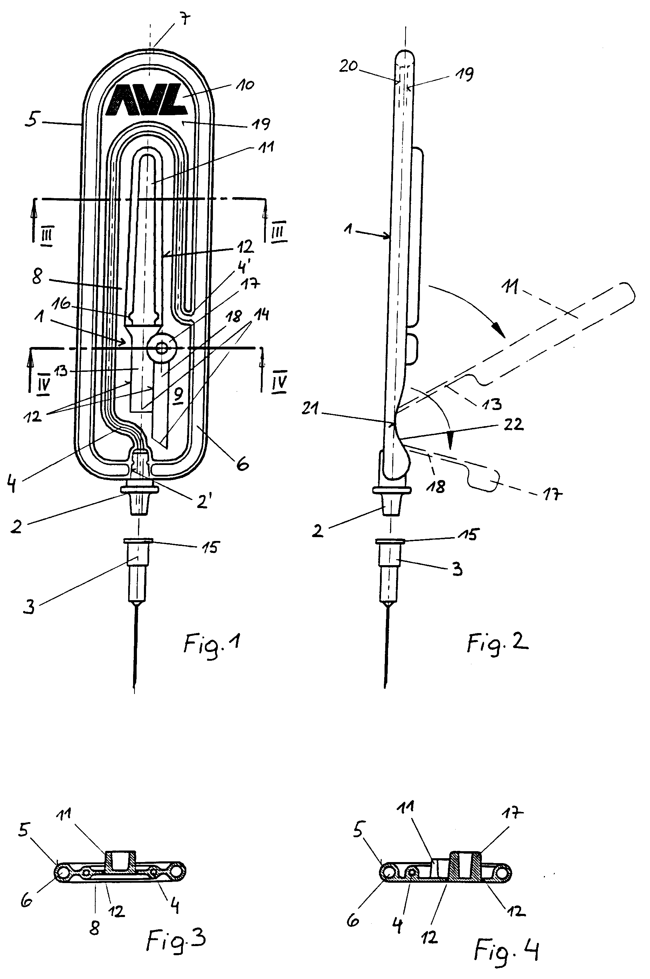

Die Erfindung wird im folgenden anhand von Zeichnungen näher erläutert. Es zeigen Fig. 1 eine Draufsicht des erfindungsgemäßen Blutentnahmegerätes, Fig. 2 eine Seitenansicht, die Fig. 3 bzw. 4 Schnittdarstellungen entlang der Linien III-III bzw. IV-IV in Fig. 1 und die Fig. 5 bzw. 6 eine Ausführungsvariante in einer Drauf- bzw. Seitenansicht gemäß Fig. 1 bzw. 2.

Das etwa in normaler Größe in den Fig. 1 bis 4 dargestellte Blutentnahmegerät (Sampler) weist einen flachen (siehe Fig. 2) blättchenförmigen Griffteil 1 in der Form eines schmalen Rechteckes mit abgerundeten Kanten auf, an welches entweder an einem Ende ein Nadeladapter 2 einstückig angeformt ist, oder welches eine Öffnung 2' aufweist, in die ein vorgeformter Nadeladapter 2 einsteckbar ist. Auf den Nadeladapter 2 kann eine herkömmliche Punktionskanüle bzw. Entnahmenadel 3 mit Hilfe z. B. eines Luer-Verschlusses aufgesteckt werden. Alle übrigen, im folgenden beschriebenen Teile des Blutentnahmegerätes sind einstückig, d. h. in einem Arbeitsgang, als Blasformteil hergestellt.

Der Griffteil 1 des in der dargestellten Variante aus durchsichtigem Kunststoff bestehenden Entnahmegerätes weist anschließend an den Nadeladapter 2 eine Probenkanüle 4 auf, welche in ein einen wulstförmigen Rand 5 des Griffteiles 1 bildendes schlauch- oder röhrchenförmiges Reservoir 6 einmündet. An der Einmündung in das Reservoir 6 kann eine Einschnürung 4' ausgebildet sein. Die Entlüftung des Blutentnahmegerätes erfolgt über eine kleine Bohrung 7 im Reservoir 6. In der dargestellten Ausführungsvariante sind die Probenkapillare 4 und das schlauchförmige Reservoir 6 so angeordnet, daß ein zentraler, einstückig mitgeformter Stegbereich 8 sowie weitere Stegbereiche 9, 10 etc. zwischen Probenkapillare 4 und Reservoir 6 zur Aufnahme bzw. Ausformung weiterer integrierter Bauteile des Blutentnahmegerätes Verwendung finden.

Im zentralen Stegbereich 8 ist ein einstückig mitgeformter Nadelprotektor 11 vorgesehen, welcher zum Stegbereich 8 Sollbruchstellen 12 aufweist, sodaß er mit leichtem Fingerdruck aus dem Griffteil 1 herausgeklappt werden kann. Eine derartige Stellung ist strichliert in Fig. 2 angedeutet. Der Nadelprotektor 11 kann über ein angeformtes biegsames Verbindungsstück 13, welches mit einem Filmscharnier am Griffteil 1 bzw. am Stegbereich befestigt ist, nach erfolgter Probennahme über die aufgesteckte Punktionskanüle 3 geklappt werden, sodaß ein Flansch 15 des Luer-Verschlusses der Punktionskanüle 3 in eine Nut 16 des Nadelprotektors 11 einrastet und diese fixiert. Der Nadelprotektor 11 wird sodann mitsamt der Punktionskanüle 3 derart um die Achse der Punktionskanüle gedreht, daß das Filmscharnier 14 bricht und so die Nadel mitsamt dem Protektor vom Blutentnahmegerät entfernt und sicher entsorgt werden kann.

In gleicher Weise kann eine integral ausgebildete Verschlußkappe 17 aus einem weiteren Stegbereich 9 des Griffteiles herausgeklappt werden, wobei auch hier Sollbruchstellen 12 und ein biegsames Verbindungsstück 18 vorgesehen sind, welches über ein weiteres Filmscharnier 14 mit dem Griffteil 1 bzw. dem Stegbereich 9 drehbar verbunden ist. Die herausgeklappte Verschlußkappe 17 ist strichliert in Fig. 2 dargestellt. Mit der Verschlußkappe 17 wird der Nadeladapter 2 nach erfolgter Probennahme und nach dem Entfernen der Punktionskanüle 3 gas- und flüssigkeitsdicht verschlossen.

Weitere Stegbereiche 10 innerhalb des wulstförmigen Randes 5 können eine glatte Oberfläche 19 aufweisen, welche für die Beklebung mit Etiketten oder maschinenlesbaren Codierungen geeignet ist. Die rückseitige Oberfläche 20 des Bereiches 10 oder auch weitere Stegbereiche können aufgerauhte, zur direkten Beschriftung z.B. mittels Faserschreiber geeignete Flächen aufweisen.

Im adapterseitigen Abschnitt des Griffteiles 1 kann durch eine Eindellung 21 des wulstförmigen Randes 5 eine Griffmulde 22 zur sicheren Handhabung des Blutentnahmegerätes vorgesehen sein.

Das Volumen der Probenkapillare 4 wird durch die Blasform, die Materialwandstärke und die Länge der Kapillare definiert. Ein typisches Volumen, z. B. für die Analyse von Blutgas- und Elektrolytmeßwerten liegt für moderne Analysatoren bei ca. 200 µl. Die Materialwandstärke für die Probenkapillare 4, das Reservoir 6 und die Stegbereiche 8 bis 10 liegt je nach verwendetem Ausgangsmaterial bei etwa 1 bis 2 mm. Für verschiedene Anwendungszwecke kann die Länge und der Durchmesser der Probenkapillare so gewählt werden, daß sich ein Probenvolumen zwischen 100 und 500 µl ergibt.

Die Schnittführung gemäß Fig. 3 im Bereich des Nadelprotektors 11 zeigt symmetrisch zum Reservoir 6 und zur Probenkapillare 4 angeordnete Stegbereiche 8. In einer Ausführungsvariante gemäß Fig. 4 (Schnitt im Bereich der Verschlußkappe 17) sind die Stegbereiche 9 einseitig an der Probenkapillare 4 und dem schlauchförmigen Reservoir 6 angeformt.

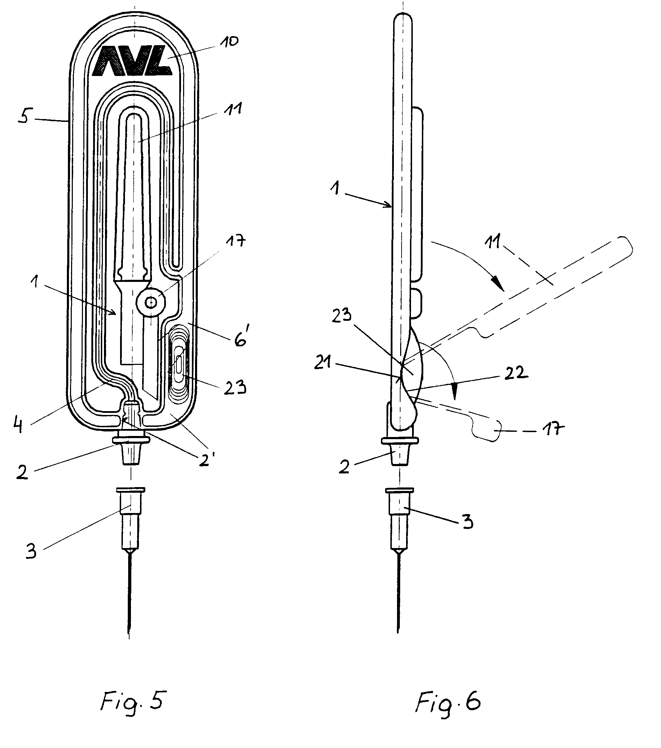

Die Ausführungsvariante nach den Fig. 5 und 6 ist in allen wesentlichen Teilen gleich aufgebaut, wie die Variante gemäß Fig. 1 und 2. Lediglich das Reservoir 6' weist hier keine Entlüftung auf, sondern einen durch Fingerdruck verformbaren Bereich 23, welcher beispielsweise im Bereich der Griffmulde 22 angeordnet sein kann. Durch eine Druckbelastung bzw. Entlastung des verformbaren Bereiches 23 kann eine Pump- bzw. Saugwirkung in der Probenkapillare 4 erzielt werden.

Die in den Fig. 1 bis 6 dargestellte Anordnung der einzelnen Bauteile eines Blutentnahmegerätes ist nur als Ausführungsbeispiel zu verstehen. Selbstverständlich fallen auch andere Anordnungen unter die erfinderische Grundidee, solange sich die wesentlichen Komponenten eines Blutentnahmegerätes, wie Probenkapillare und integrierter Griffteil sowie ggf. der Nadeladapter und ein Reservoir einstückig als Blasformteil herstellen lassen.

## Patentansprüche

1. Blutentnahmegerät, insbesondere zur Entnahme von arteriellen Blutproben, mit einem Griffteil (1), einem Nadeladapter (2), auf welchem eine Punktionskanüle (3) aufsteckbar ist, sowie einer mit der Punktionskanüle (3) in Verbindung stehenden Probenkapillare (4), **dadurch gekennzeichnet**, daß der Griffteil (1) und die Probenkapillare (4) einstückig als Blasformteil ausgeführt sind und daß der Nadeladapter (2) entweder einstückig mitgeformt ist oder als separater Bauteil in eine in die Probenkapillare (4) mündende Öffnung (2') des Griffteiles (1) einsteckbar ist.

2. Blutentnahmegerät nach Anspruch 1, **dadurch gekennzeichnet**, daß an das vom Nadeladapter (2) abgewandte Ende der Probenkapillare (4) ein einstückig mitgeformtes, mit einer Entlüftung (7) versehenes Reservoir (6) anschließt, welches als Probenüberlauf dient.

3. Blutentnahmegerät nach Anspruch 2, **dadurch gekennzeichnet**, daß das an die Probenkapillare (4) anschließende Reservoir (6) schlauch- oder röhrchenförmig ausgebildet ist, und in einem wulstförmigen Rand (5) des im wesentlichen plättchenförmigen Griffteiles (1) integriert ist.

4. Blutentnahmegerät nach Anspruch 1, **dadurch gekennzeichnet**, daß an das vom Nadeladapter (2) abgewandte Ende der Probenkapillare (4) ein einstückig mitgeformtes Reservoir (6') anschließt, welches einen durch Fingedruck verformbaren Bereich (23) zum Füllen und Entleeren der Probenkapillare (4) aufweist.

5. Blutentnahmegerät nach einem der Ansprüche 1 bis 4, **dadurch gekennzeichnet**, daß der Griffteil (1) einen einstückig mitgeformten Nadelprotektor (11) aufweist, welcher über ein mitgeformtes Drehscharnier, vorzugsweise ein Filmscharnier (14), am Griffteil (1) befestigt ist, wobei der Nadelprotektor (11) durch seine Formgebung zur sicheren Aufnahme der Punktionskanüle (3) ausgebildet ist.

6. Blutentnahmegerät nach einem der Ansprüche 1 bis 5, **dadurch gekennzeichnet**, daß der Griffteil (1) eine einstückig mitgeformte Verschlußkappe (17) aufweist, welche über ein mitgeformtes Drehscharnier, vorzugsweise ein Filmscharnier (14), am Griffteil (1) befestigt ist, wobei die Verschlußkappe (17) durch ihre Formgebung zum sicheren Verschluß des Nadeladapters (2) ausgebildet ist.

7. Blutentnahmegerät nach Anspruch 5 oder 6, mit einem im wulstförmigen Rand (5) des Griffteiles (1) integrierten schlauch- oder röhrchenförmigen Reservoir (6, 6'), **dadurch gekennzeichnet**, daß die Längsachsen des schlauch- oder röhrchenförmigen Reservoirs (6, 6') und der Probenkapillare (4) im wesentlichen in der Ebene des plättchenförmigen Griffteiles (1) oder in einer Parallelebene dazu liegen, sowie daß der Griffteil (1) hauptsächlich durch einstückig mitgeformte Stegbereiche (8, 9, 10) gebildet wird.

8. Blutentnahmegerät nach Anspruch 7, **dadurch gekennzeichnet**, daß ein zentraler Stegbereich (8) für die integrale Ausbildung des Nadelprotektors (11) vorgesehen ist, welcher an seinem Umfang über mitgeformte, leicht durchdrückbare Sollbruchstellen (12) und an einem biegsamen Verbindungsstück (13) über das Filmscharnier (14) mit dem Griffteil (1) in Verbindung steht.

9. Blutentnahmegerät nach Anspruch 7 oder 8, **dadurch gekennzeichnet**, daß ein weiterer Stegbereich (9) für die integrale Ausbildung der Verschlußkappe (17) vorgesehen ist, welche an ihrem Umfang über mitgeformte, leicht durchdrückbare Sollbruchstellen (12) und an einem biegsamen Verbindungsstück (18) über das Filmscharnier (14) mit dem Griffteil (1) in Verbindung steht.

10. Blutentnahmegerät nach einem der Ansprüche 7 bis 9, **dadurch gekennzeichnet**, daß weitere Stegbereiche (10) vorgesehen sind, welche eine für die direkte Beschriftung des Blutentnahmegerätes geeignete, vorzugsweise aufgerauhte Oberfläche (20) aufweisen.

11. Blutentnahmegerät nach einem der Ansprüche 7 bis 10, **dadurch gekennzeichnet**, daß weitere Stegbereiche (10) vorgesehen sind, welche eine für die Beklebung mit Etiketten oder maschinenlesbaren Codierungen geeignete, vorzugsweise glatte Oberfläche (19) aufweisen.

12. Blutentnahmegerät nach einem der Ansprüche 7 bis 11, **dadurch gekennzeichnet**, daß im adapterseitigen Abschnitt des Griffteiles (1) durch eine Eindellung (21) des wulstförmigen Randes (5) eine Griffmulde (22) zur sicheren Handhabung des Blutentnahmegerätes vorgesehen ist.

13. Blutentnahmegerät nach einem der Ansprüche 1 bis 12, **gekennzeichnet durch** die Verwendung von Plastikmaterial, vorzugsweise von z. B. Acrylnitril-Methyl-Acrylat-Copolymerisate, PMMA, Polycarbonat, Polyethylen und Polypropylen, welches für Blutgase wie O₂ und CO₂ eine möglichst geringe Permeabilität aufweist.

14. Blutentnahmegerät nach einem der Ansprüche 1 bis 13, **gekennzeichnet durch** die Verwendung von durchsichtigem Material.

15. Verfahren zur Herstellung eines Blutentnahmegerätes, vorzugsweise zur Entnahme von arteriellem Blut, nach einem der Ansprüche 1 bis 14, **dadurch gekennzeichnet,** daß das Blutentnahmegerät aus einem thermoplastisch verformbaren, schlauchförmigen Grundkörper im wesentlichen einstückig als Blasformteil hergestellt wird, wobei die äußeren Konturen durch ein vorzugsweise zweiteiliges Preßwerkzeug und die Lumen der Probenkapillare und allfälliger weiterer Kanäle durch Blasformen dargestellt werden.

16. Verfahren nach Anspruch 15, **dadurch gekennzeichnet**, daß die Innenoberfläche des Blutentnahmegerätes mit einer Heparinlösung, vorzugsweise mit in destilliertem Wasser gelöstem Heparin, gespült werden.
